# EUROPEAN PATENT APPLICATION

(11) **EP 3 957 285 A1**
(43) Date of publication of application: **23.02.2022**
(21) Application number: 20791271.8
(22) Date of filing: 15.04.2020
(51) Int. Cl.: A61F 13/00, A61F 13/02

(54) **COVERING FILM, SYSTEM COMPRISING SAME, AND USAGE METHOD AND APPLICATION THEREFOR**

(30) Priority: 15.04.2019 CN 201910300866; 15.04.2019 CN 201920508226 U
(71) Applicant: Hozhou Innovation Pharmaceutical Co., Ltd., Huzhou, Zhejiang 313001 (CN)
(72) Inventor: ZHANG, Jie, Huzhou, Zhejiang 313001 (CN); TANG, Peike, Huzhou, Zhejiang 313001 (CN)
(74) Representative: Dehns
(86) International application number: PCT/CN2020/084858
(87) International publication number: WO 2020/211763

(57) **Abstract**

A medical covering film and system, and a usage method and an application therefor. The medical covering film comprises a barrier film layer (10), an adsorption layer (20), and a reticular adhesive layer (30), one side of the adsorption layer (20) being composited on the barrier film layer (10), and the reticular adhesive layer (30) being composited on the other side of the adsorption layer (20); the area of an adhesive-free region (31) of the reticular adhesive layer (30) occupies 10% or more of the total area of the reticular adhesive layer (30), but not 100%. The usage method is as follows: coating a water-containing semi-solid preparation (40) on a central region of the reticular adhesive layer (30) of the medical covering film; covering affected skin with the medical covering film so that the preparation comes into contact with the affected skin; and then adhering an edge region of the reticular adhesive layer (30) to skin outside of the affected skin. The present medical covering film can fix a preparation to the affected skin very well, is not easily squeezed off the target skin area by external force, and can maintain the moisture in the preparation very well.

## Description

The present application claims the priority to Chinese Patent Application CN201910300866.7 and CN201920508226.0 filed on April 15, 2019, the content of which is incorporated herein by reference in its entirety.

### Technical Field

The present disclosure relates to a covering film, a system comprising the same, and usage method and application therefor.

### Background

In many disease treatments and skin nutrition applications, water-containing semi-solid preparations (such as gels, emulsions) ideally need to be fixed and sealed on the skin surface for a long time.

For example, for controlling postherpetic neuralgia, a semi-solid and water-containing (such as gel, cream) local anesthetic preparation needs to be kept on the affected skin for more than 6 hours, or even more than 12 hours, to obtain a satisfactory duration of analgesic effect for the patient. During such a long period of administration, the local anesthetic preparation must not flow away from the affected skin area, and the evaporation of water from the local anesthetic preparation must also be controlled to be below a certain amount. Otherwise, the duration of the effects of local anesthetics will be greatly reduced.

In order to keep the local anesthetic preparation on the affected skin area, the existing method is to cover the local anesthetic preparation layer applied on the skin with a plastic film (such as cling film) (for example, EMLA cream used for skin anesthesia before surgery, the usual usage method thereof is covering the cream with plastic film for 1-1.5 hours).

However, in many cases, it is necessary to keep the water-containing semi-solid preparation on the affected skin for more than 6 hours or even more than 12 hours, and there are serious problems with the method of covering with plastic film: (1) the plastic film does not have the ability to fix and adsorb the water-containing semi-solid preparation, so the covered water-containing semi-solid preparation would leave the original place where it was originally applied under the action of gravity or squeeze; (2) the plastic film itself does not have the ability to be fixed on the skin, so another tape must be used to fix the plastic film on the skin; (3) the boundary of the water-containing semi-solid preparation covered by the plastic film is in communication with the outside air, so the moisture in the water-containing semi-solid preparation will evaporate. When the water in the water-containing semi-solid preparation is lower than a certain value, the transdermal absorption rate of its active ingredients may be greatly reduced. If a water-containing semi-solid preparation is covered with an adhesive film fully covered with adhesive, the part of the adhesive film that is in contact with the water-containing semi-solid preparation cannot absorb the water-containing semi-solid preparation, so the water-containing semi-solid preparation will also leave the affected skin under the action of external force (such as gravity or squeeze). Therefore, covering the semi-solid local anesthetic preparation with a plastic film or an adhesive film fully covered with adhesive is not ideal.

Many water-containing semi-solid preparations, such as preparations for the treatment of skin diseases, preparations for joint muscle inflammation, and preparations for nourishing the skin, need to be fixed and sealed on the skin for a long time.

### Content of the present invention

The technical problem to be solved in the present disclosure is to overcome the defeats of the prior art that the method of covering with plastic film or plastic adhesive film cannot fix the water-containing semi-solid preparation on the skin well, and cannot retain the moisture in the preparation, and to provide a novel type of covering film, a system comprising the same, a usage method and a use thereof. The covering film can fix the water-containing semi-solid preparation on the affected skin very well, is not easily squeezed off the target skin area by external force, and can maintain the moisture in the water-containing semi-solid preparation very well.

The present disclosure solves the above-mentioned technical problems through the following technical solutions:

The present disclosure provides a covering film, the covering film comprises a barrier film layer and an adsorption layer.

In the present disclosure, the "covering film" includes a "medical covering film", when the covering film is used for medical purposes, those skilled in the art know that a medical covering film is used.

In the covering film (for example, medical covering film), preferably, the covering film (for example, medical covering film) contains a cross-linking agent. The cross-linking agent is preferably dispersed in the material of the adsorption layer.

Preferably, the covering film (for example, medical covering film) contains a cross-linking agent higher than 0.01 mg/per square centimeter, more preferably higher than 0.1 mg/per square centimeter, or 0.01 mg-100 mg/per square centimeter, or 0.02-0.2 mg/per square centimeter.

Preferably, the cross-linking agent is a substance containing boron element (such as sodium borate), glutaraldehyde, glyoxal, maleic acid, citric acid, trisodium trimetaphosphate, sodium hexametaphosphate, dianhydride, succinic acid, sulfosuccinic acid, or N,N'-methylenebisacrylamide.

When the covering film (for example, medical covering film) contains a cross-linking agent, the purpose is to achieve a better use effect of the covering film in the following use scenarios: the covering film covers/wraps the skin surface (with or without a wound) coated with a semi-solid preparation layer, the preparation layer contains a cross-linkable substance (if the cross-linkable substance is adhesive agent, it can be called a cross-linkable adhesive agent). After the cross-linking agent diffuses from the covering film into the preparation layer, the cross-linking agent can undergo a cross-linking reaction with the corresponding "cross-linkable substance" in the preparation layer to solidify the preparation layer.

After the covering film (for example, medical covering film) covers the preparation layer, the following processes occur: the cross-linking agent (for example, substance containing boron element) in the covering film diffuses into the preparation, causing a cross-linking reaction with cross-linkable substances in the preparation (such as polyvinyl alcohol), thereby causes the preparation to solidify and adhere to the covering film (for example, medical covering film). In this way, when the scheduled administration time is over, the covering film (for example, medical covering film) can be removed and the preparation that has been solidified and adhered to the covering film (for example, medical covering film) can be removed simultaneously. In this way, there is no residual preparation on the skin, and the patient does not need to wash the skin.

Preferably, the cross-linking agent is a substance containing boron element, such as sodium borate; in this case, the preparation preferably contains polyvinyl alcohol.

Preferably, the cross-linking agent is N,N'-methylenebisacrylamide, in this case, the preparation contains polyvinylpyrrolidone.

When the cross-linking agent is a substance containing boron element, the covering film contains a substance containing boron element preferably higher than 0.01 mg/per square centimeter, more preferably higher than 0.1 mg/per square centimeter, or 0.01 mg -100 mg/per square centimeter, or 0.02-2 mg/per square centimeter; at this time, the mass percentage of polyvinyl alcohol in the preparation is preferably 0.2-30%, more preferably 0.5-15%, and further more preferably 1%-5%.

In the covering film (for example, medical covering film), the covering film (for example, medical covering film) further comprises a reticular adhesive layer, one side of the adsorption layer is composited on the barrier film, and the reticular adhesive layer is composited on the other side of the adsorption layer; the area of the adhesive-free region of the reticular adhesive layer occupies 10% or more of the total area of the reticular adhesive layer, but not 100%.

In the covering film (for example, medical covering film), the barrier film layer is a barrier film conventionally used in the medical field, and the material of the barrier film may be a material impermeable to water vapor or a material with limited water vapor permeability, such as polyethylene film, ethylene-vinyl acetate copolymer film (referred to as EVA film) or polyurethane film.

In the covering film (for example, medical covering film), the adsorption layer may be a material capable of adsorbing or retaining a preparation, for example, a water-containing semi-solid preparation in the prior art, for example, a non-woven fabric with strong absorptivity.

In the covering film (for example, medical covering film), the reticular adhesive layer only needs to have an adhesive region and an adhesive-free region. For example, the adhesive region of the reticular adhesive layer is an adhesive net composed of curves and/or straight lines. Preferably, the adhesive region of the reticular adhesive layer is an adhesive net composed of a set of parallel longitude lines and a set of parallel latitude lines. More preferably, the longitude lines and the latitude lines are perpendicular to each other. Even more preferably, the distance between two adjacent longitude lines is equal to the distance between two adjacent latitude lines. Further more preferably, the width of each of the longitude lines and each of the latitude lines is 0.5 mm-1 mm. Here, it should be noted that the longitude lines and the latitude lines on the reticular adhesive layer constitute the adhesive region, and the other regions on the reticular adhesive layer except the longitude lines and the latitude lines constitute the adhesive-free region.

In the covering film (for example, medical covering film), the thickness of the reticular adhesive layer is preferably 0.01 mm-0.75 mm, more preferably 0.05 mm-0.75 mm, even more preferably 0.25 mm-0.75 mm.

In the covering film (for example, medical covering film), the adhesive of the reticular adhesive layer may be a medical pressure-sensitive adhesive that is insoluble in water, preferably silicone pressure-sensitive adhesive and/or acrylic pressure-sensitive adhesive.

In the covering film (for example, medical covering film), one side of the adsorption layer may be adhered to the barrier film layer by adhesive, or composited on the barrier film layer by hot pressing.

In the covering film (for example, medical covering film), the reticular adhesive layer may be adhered to the other side of the adsorption layer by using existing spray coating method.

In the covering film (for example, medical covering film), preferably, the area of the adhesive-free region of the reticular adhesive layer occupies 30% or more of the total area of the reticular adhesive layer. More preferably, the area of the adhesive-free region of the reticular adhesive layer occupies 50% or more of the total area of the reticular adhesive layer. Even more preferably, the area of the adhesive-free region of the reticular adhesive layer occupies 70% or more of the total area of the reticular adhesive layer, for example, it may be 70%-90%.

In the covering film (for example, medical covering film), preferably, the length stretch rate of the covering film (for example, medical covering film) in all directions is more than 10%, for example, it may be 10%-30%. The covering film (for example, medical covering film) with the stretch rate will be more comfortable when used in parts such as joints and muscles.

The present disclosure also provides a system for fixing the preparation on the skin, the system comprises the covering film (for example, medical covering film) and a preparation layer for coating on the affected skin; the covering film (for example, medical covering film) is used to cover the affected skin area coated with the preparation layer in a way that the reticular adhesive layer orients towards the affected skin; the central region of the reticular adhesive layer is used for contacting the preparation layer; the edge region of the reticular adhesive layer is used to adhere to the skin area beyond the affected skin or the preparation layer, so as to fix the preparation layer on the affected skin or the skin beyond the affected skin, and the preparation layer is completely enclosed in a closed space composed of the covering film (for example, medical covering film) and the skin beyond the affected skin or the skin.

In the system, the preparation is preferably a water-containing semi-solid preparation.

The system above can achieve two purposes simultaneously: 1. the system can make the preparation (for example, water-containing semi-solid preparation) contact with the adsorption layer of the covering film (for example, medical covering film), so that the preparation (for example, water-containing semi-solid preparation) is not easy to be squeezed off the target skin area by external force; 2. the covering film (for example, medical covering film) of the system covers the preparation (for example, water-containing semi-solid preparation), thus retaining the moisture in the preparation (for example, water-containing semi-solid preparation) and fixing the preparation (for example, water-containing semi-solid preparation) on the skin. By contrast, the covering material in the prior art cannot achieve the above-mentioned two purposes simultaneously: if a commonly used plastic adhesive film (that is, a medical film coated with adhesive) is used, the above-mentioned second purpose can be achieved but the above-mentioned first purpose cannot be achieved; if gauze is used, the above-mentioned first purpose can be achieved but the above-mentioned second purpose cannot be achieved.

In the above system, the water-containing semi-solid preparation refers to a preparation that contains water and has a viscosity greater than water but is not completely solid. The water-containing semi-solid preparation may be a gel, a cream or a viscous liquid; or, the water-containing semi-solid preparation may be a skin nourishing preparation or a local anesthetic preparation.

In the system, preferably, the length of the outer edge of the covering film (for example, medical covering film) away from the outer edge of the preparation layer (for example, water-containing semi-solid preparation layer) is more than 5 mm, for example, 2 cm- 4 cm.

The present disclosure also provides a system for fixing the preparation on the skin, the system comprises the covering film (for example, medical covering film); wherein, the covering film (for example, medical covering film) comprises a barrier film layer and an adsorption layer, but does not comprise a reticular adhesive layer; the cross-linking agent is dispersed in the material of the adsorption layer; the system also comprises a preparation layer for coating on the affected skin; wherein, the preparation contains an adhesive agent and/or a cross-linkable substance.

In the system, the preparation is preferably a water-containing semi-solid preparation.

The preferred and more preferred solutions of "the water-containing semi-solid preparation" and "the length of the outer edge of the covering film (for example, medical covering film) away from the outer edge of the preparation layer (for example, water-containing semi-solid preparation layer)" are as described above.

In the system, when the cross-linkable substance is adhesive agent, it can be called a cross-linkable adhesive agent.

Preferably, the preparation contains a cross-linkable substance and the covering film (for example, medical covering film) contains a corresponding cross-linking agent.

More preferably, the cross-linkable substance is polyvinyl alcohol, the corresponding cross-linking agent is sodium borate.

More preferably, the cross-linkable substance is polyvinylpyrrolidone, the corresponding cross-linking agent is N,N'- methylenebisacrylamide.

When the preparation contains adhesive agent, the covering film (for example, medical covering film) can be fixed on the preparation layer, and no additional fixing method is required. Specifically, after the covering film (for example, medical covering film) contacts the preparation layer, the cross-linking agent in the covering film (for example, medical covering film) diffuses into the preparation layer, and undergoes a cross-linking reaction with the cross-linkable substance therein, solidifying the preparation layer and causing the solidified preparation layer to adhere to the covering film (for example, medical covering film).

Therefore, when using this system, it is necessary to wait for sufficient time for the solidification process to be completed. When using this system, the preparation is not completely enclosed in a closed space, because the edges of the preparation layer are not sealed by the reticular adhesive layer and the skin as some of the aforementioned systems. Theoretically, the edge portion of the preparation layer (for example, water-containing semi-solid preparation layer) will lose part of the moisture contained therein due to evaporation, which may affect the absorption of the drug. However, for a relatively large area of the preparation layer, the decrease in drug absorption in a small part of the edge region will not affect its overall efficacy. The covering film (for example, medical covering film) without the reticular adhesive layer is easier to manufacture and has lower cost.

The present disclosure also provides a usage method of the above-mentioned covering film (for example, medical covering film), the usage method comprises the following steps: applying a preparation (for example, water-containing semi-solid preparation) on the central region of the reticular adhesive layer of the covering film (for example, medical covering film), so that the edge region of the reticular adhesive layer is not coated by the preparation (for example, water-containing semi-solid preparation), to obtain the covering film (for example, medical covering film) with a preparation layer (for example, water-containing semi-solid preparation layer); then covering the covering film (for example, medical covering film) with the preparation layer (for example, water-containing semi-solid preparation layer) on the affected skin area, and making the preparation layer (for example, water-containing semi-solid preparation layer) in contact with the affected skin area, the edge region is adhered to the skin beyond the affected skin or the skin not covered by the preparation layer (for example, water-containing semi-solid preparation layer), so as to fix the preparation layer (for example, water-containing semi-solid preparation layer) on the affected skin or the skin beyond the affected skin, and the preparation layer (for example, water-containing semi-solid preparation layer) is completely enclosed in a closed space composed of the covering film (for example, medical covering film) and the skin or the skin beyond the affected skin.

The usage method, like the systems, can achieve the above two purposes simultaneously. By contrast, none of the methods used in the prior art can achieve the above two purposes simultaneously.

In the usage method, the preparation (for example, water-containing semi-solid preparation) is completely enclosed in the closed space. When the barrier film used in the barrier film layer is a material impermeable to water vapor, the moisture in the preparation (for example, water-containing semi-solid preparation) will not evaporate, so that the moisture in the preparation

(for example, water-containing semi-solid preparation) can be well retained. When the barrier film used in the barrier film layer has a certain water vapor permeability rate, the moisture in the preparation (for example, water-containing semi-solid preparation) will evaporate, at this time, the water vapor permeability of the barrier film can be reasonably selected according to the moisture content of the preparation (for example, water-containing semi-solid preparation) and the required usage time, the barrier film selected according to the above principles will ensure that the rate of transdermal absorption of the effective ingredients in the preparation (for example, water-containing semi-solid preparation) is not affected within the required usage time.

The above-mentioned usage method can also be carried out as following steps: the covering film (for example, medical covering film) is covered on the affected skin coated with the preparation layer (for example, water-containing semi-solid preparation layer) and surrounding skin in a way that the reticular adhesive layer orients towards the affected skin; the central region of the reticular adhesive layer contacts the preparation layer (for example, water-containing semi-solid preparation layer); the edge region of the reticular adhesive layer adheres to the skin beyond the affected skin or the skin not covered by the preparation layer, so as to fix the preparation layer (for example, water-containing semi-solid preparation layer) on the affected skin or the skin beyond the affected skin, and the preparation layer (for example, water-containing semi-solid preparation layer) is completely enclosed in a closed space composed of the covering film (for example, medical covering film) and the skin beyond the affected skin or the skin.

Here, it should be noted that those skilled in the art should know that when the covering film (for example, medical covering film) is used, the reticular adhesive layer of covering film (for example, medical covering film) orients towards the affected skin, and after the covering film (for example, medical covering film) is adhered to the skin beyond the affected skin or the skin beyond the area covered by the preparation layer, the covering film (for example, medical covering film) can completely cover the preparation layer (a water-containing semi-solid preparation layer), and the central region of the reticular adhesive layer is in contact with the preparation layer (a water-containing semi-solid preparation layer). At the same time, the edge region of the reticular adhesive layer that is not in contact with the preparation (for example, water-containing semi-solid preparation) and is adhered to the skin beyond the affected skin or the skin not covered by the preparation layer, so that the preparation (for example, water-containing semi-solid preparation) can be fixed to the skin.

In the usage method, preferably, after the covering film (for example, medical covering film) is adhered to the skin beyond the affected skin or the skin not covered by the preparation layer, the distance or the length of the outer edge of the covering film (for example, medical covering film) away from the outer edge of the preparation layer (for example, water-containing semi-solid preparation layer) is more than 5 mm, for example, 2 cm-4 cm.

In the usage method, the water-containing semi-solid preparation refers to a preparation that contains water and has a viscosity greater than water but is not completely solid. The water-containing semi-solid preparation may be a gel, a cream or a viscous liquid; or, the water-containing semi-solid preparation may be a skin nourishing preparation or a local anesthetic preparation.

In the system and the usage method, the central region refers to the region on the reticular adhesive layer that is not in contact with the preparation layer, and the edge region refers to the region on the reticular adhesive layer that is not the central region.

The present disclosure also provides an application of the covering film (for example, medical covering film) in the preparation of a medical device for treating herpes zoster pain, post herpetic neuralgia, neuroma pain, phantom limb pain, diabetic peripheral neuralgia, joint pain, osteoarthritis pain, back pain, pain caused by gout, pain caused by soft tissue injury, postoperative incision pain, pain of broken skin caused by disease or trauma, burn pain or pain during burn scab removal.

The present disclosure also provides a usage method of the system for fixing the preparation on the skin, wherein, the preparation is applied to the affected skin to form a preparation layer; and the covering film (for example, medical covering film) is covered on the preparation layer, let the cross-linking agent and the cross-linkable substance undergo a cross-linking reaction to solidify the preparation layer.

Wherein, the solidification of the preparation layer comprises the entire process of allowing the cross-linking agent in the covering film (for example, medical covering film) to diffuse into the preparation layer, and cross-linking reaction undergone with the cross-linkable substance in the preparation layer.

In the present disclosure, "affected skin" refers to the skin area of a human body that, when covered by the pharmaceutical preparation, can bring therapeutic effect. For example, the affected skin area of the herpes zoster is the skin of the herpes area, the affected skin of joint pain is the skin covering the joint and adjacent to the joint, and affected skin of the surgical incision is the skin of the surgical incision itself and the skin adjacent thereto.

In the present disclosure, skin comprises intact skin, mucous membranes, and damaged skin and mucous membranes caused by disease or trauma.

Advantageous effects of the present disclosure are: the present disclosure provides a covering film, a system comprising the same, and a usage method and a use thereof. The covering film can fix the preparation on the affected skin area very well, the preparation is not easily squeezed off the target skin area by external force, and the covering film can maintain the moisture in the preparation very well.

### Brief description of the drawings

Fig.1 is a schematic diagram of the structure of each layer of the medical covering film of Examples 1-6;
Fig.2 is a schematic diagram of the structure of the medical covering film of Examples 1-6;
Fig.3 is a top view of the medical covering film coated with a water-containing semi-solid preparation layer;
Fig.4 is a cross-sectional view of the medical covering film covering a water-containing semi-solid preparation layer in Application Examples 1-4 in the state of usage;
Fig.5 is a schematic diagram of the structure of the medical covering film of Example 7.

### Description of reference signs in the drawings:

barrier film layer 10
adsorption layer 20
non-woven fabric layer containing sodium borate 201
reticular adhesive layer 30
adhesive-free region 31
adhesive region 32
water-containing semi-solid preparation layer 40

### Detailed description of the preferred embodiment

The present disclosure will be further explained below by means of examples, but the present disclosure is not limited to the scope of the described examples. In the following examples, the experimental methods without specific conditions are selected according to conventional methods and conditions, or according to the commodity specifications.

**Table 1 material and structural parameters of the medical covering film for each example**

| Components | | Example | | | | | | |
|---|---|---|---|---|---|---|---|---|
| | | 1 | 2 | 3 | 4 | 5 | 6 | 7 |
| Barrier film layer 10 | Material | polyethyle ne film | EVA film | polyuretha ne film | polyuretha ne film | polyethyle ne film | polyuretha ne film | polyethyle ne film |
| Adsorptio n layer 20 | Material | non-woven fabric | non-woven fabric | non-woven fabric | non-woven fabric | non-woven fabric | non-woven fabric | non-woven fabric |
| Reticular adhesive layer 30 | Type of adhesive | silicone pressure-sensitive adhesive | acrylic pressure-sensitive adhesive | silicone pressure-sensitive adhesive | acrylic pressure-sensitive adhesive | silicone pressure-sensitive adhesive | acrylic pressure-sensitive adhesive | silicone pressure-sensitive adhesive |
| | Thickness | 0.25mm | 0.05mm | 0.5mm | 0.1mm | 0.75mm | 0.75mm | 0.025mm |
| | Width of longitude line/mm | 0.5 | 0.5 | 0.5 | 0.5 | 0.5 | 1 | 0.5 |
| | Width of latitude line/mm | 0.5 | 0.5 | 0.5 | 0.5 | 0.5 | 1 | 0.5 |
| | Side length a /mm | 0.23 | 0.60 | 1.20 | 2.60 | 4 | 9 | 0.23 |
| | Proportion b | 10% | 30% | 50% | 70% | 64% | 81% | 10% |

In Table 1, the side length a refers to the side length of each square constituting the adhesive-free region 31; proportion b refers to the proportion of the area of the adhesive-free region 31 to the total area of the reticular adhesive layer 30.

The raw materials of the water-containing semi-solid preparation (Formulation A, B, C, D) used in the following examples are shown in the following table, and the preparation method thereof is to mix the components evenly.

**Table 2 the raw materials of Formulation A**

| Components | Mass percentage or molar concentration |
|---|---|
| Lidocaine | 5wt% |
| Disodium hydrogen phosphate | 0.094 mol/L |
| Sodium dihydrogen phosphate | 0.006 mol/L |
| Xanthan gum | 4wt % |
| Sodium hydroxide | 0.08wt% |
| Water | Added to 100wt% |

**Table 3 the raw materials of Formulation B**

| Components | Mass percentage or molar concentration |
|---|---|
| Lidocaine | 8wt% |
| Disodium hydrogen phosphate | 0.094 mol/L |
| Sodium dihydrogen phosphate | 0.006 mol/L |
| Sodium hydroxide | 0.08wt% |
| Hydroxyethyl cellulose | 3wt% |

| Glycerin | 15wt% |
|---|---|
| Water | Added to 100wt% |

**Table 4 the raw materials of Formulation C**

| Components | Mass percentage |
|---|---|
| Diclofenac sodium | 2wt% |
| Ethanol | 20wt% |
| Hydroxyethyl cellulose | 3wt% |
| Glycerin | 15wt% |
| Water | Added to 100wt% |

**Table 5 the raw materials of Formulation D**

| Components | Mass percentage |
|---|---|
| Xanthan gum | 2wt% |
| Hydroxyethyl cellulose | 3wt% |
| Glycerin | 35wt% |
| Water | Added to 100wt% |

**Table 6 the raw materials of Formulation F**

| Components | Mass percentage or molar concentration |
|---|---|
| Bupivacaine | 8wt% |
| Disodium hydrogen phosphate | 0.094 mol/L |
| Sodium dihydrogen phosphate | 0.006 mol/L |
| Sodium hydroxide | 0.08wt% |
| Hydroxyethyl cellulose | 3wt% |
| Polyvinyl alcohol | 10wt% |
| Water | Added to 100wt% |

**Table 7 the raw materials of Formulation G**

| Components | Mass percentage |
|---|---|
| Diclofenac sodium | 2wt% |
| Ethanol | 20wt% |
| Hydroxyethyl cellulose | 3wt% |
| Polyvinyl alcohol | 5wt% |
| Water | Added to 100wt% |

### Examples 1-7

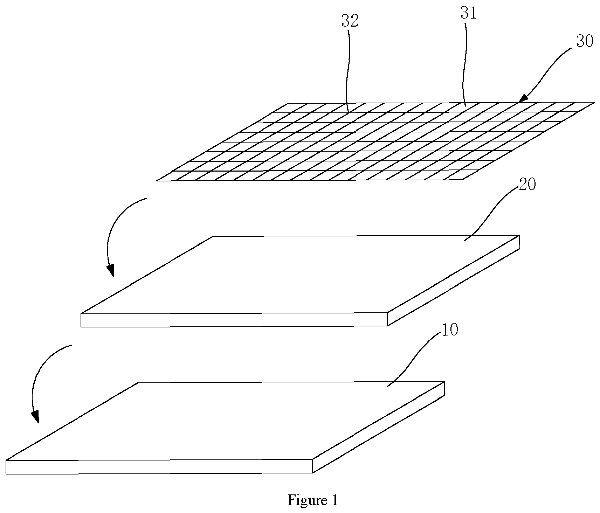

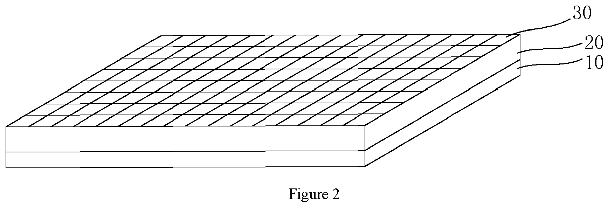

The medical covering film as shown in Fig.1 and Fig.2, the medical covering film comprises a barrier film layer 10, an adsorption layer 20, and a reticular adhesive layer 30, the adsorption layer 20 is composited on the barrier film layer 10, and the reticular adhesive layer 30 is composited on the adsorption layer 20.

Wherein, the reticular adhesive layer 30 is composed of a set of parallel longitude lines and a set of parallel latitude lines, the longitude lines and the latitude lines are perpendicular to each other, and the distance between two adjacent longitude lines is equal to the distance between two adjacent latitude lines. The longitude lines and the latitude lines on the reticular adhesive layer 30 constitute the adhesive region 32, and the other regions on the reticular adhesive layer 30 except for the longitude lines and latitude lines constitute adhesive-free region 31.

Wherein, the adsorption layer 20 is adhered to the barrier film layer 10 by adhesive, and the reticular adhesive layer 30 is adhered to the adsorption layer 20 by spray coating method.

Wherein, the material of the barrier film layer 10 and the material of the adsorption layer 20, the type of the adhesive of the reticular adhesive layer 30, the thickness of the reticular adhesive layer 30, the proportion of the area of the adhesive-free region 31 of the reticular adhesive layer 30 to the total area of the reticular adhesive layer 30 are shown in Table 1.

### Example 8

A medical covering film and a water-containing semi-solid preparation

In this example, the raw materials of the water-containing semi-solid preparation (Formulation E) used are shown in the following table, and the preparation method thereof is to mix the components evenly.

**Table 8 the raw materials of the Formulation E**

| Components | Mass percentage |
|---|---|
| Lidocaine | 5% |
| Polyvinyl alcohol | 7.5% |
| Sodium hydroxide | 0.64% |
| Xanthan gum | 1.6% |
| Water | Added to 100wt% |

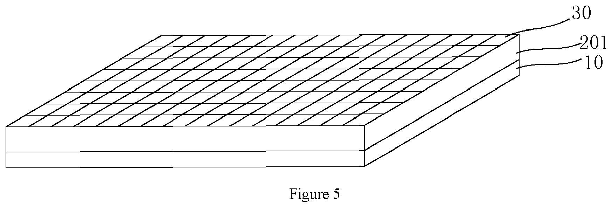

The material and structural parameters of the medical covering film are the same as those in Example 1. Additionally, the non-woven fabric contains 0.2 mg of sodium borate, 1 mg of glycerin, and 1 mg of maltodextrin per square centimeter (as shown in Fig.5). When the medical covering film is covered on a layer of the Formulation E applied to the affected skin, the sodium borate in the medical covering film diffuses into the preparation layer and undergoes cross-linking reaction with the polyvinyl alcohol in the preparation layer, solidifying and adhering Formulation E to the medical covering film. In this way, when the scheduled administration time is over, the medical covering film can be removed and Formulation E that has been solidified and adhered to the medical covering film can be removed simultaneously. In this way, there is no residual water-containing semi-solid preparation on the skin, so the patient does not need to wash the skin.

### Application Example 1

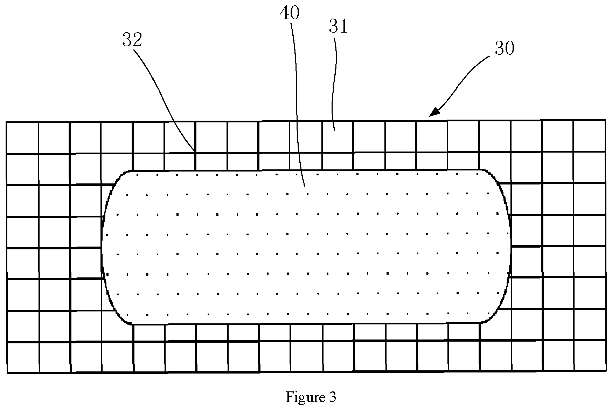

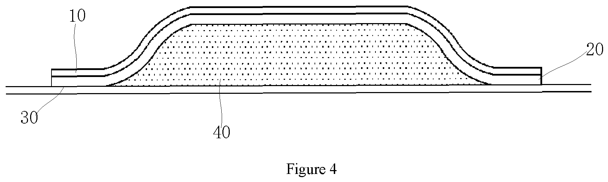

The usage method of the medical covering film of the Example 4 is as following: as shown in Fig.3, a layer of Formulation A with a thickness of 2 mm was applied on a 10 cm×10 cm of the reticular adhesive layer 30 of the medical covering film of Example 4, and Formulation A covered a 6 cmx6 cm of region of the central region of the reticular adhesive layer 30. This medical covering film with the Formulation A adhered to the skin on the back of a human subject, the Formulation A and the 2 cm wide of edge region around the reticular adhesive layer 30 were directly contacted the skin, the edge region of the reticular adhesive layer 30 formed a flat square closed space with the skin, the 6 cm×6 cm of the Formulation A layer (that is, the water-containing semi-solid preparation layer 40 shown in Fig.4) in the middle was enclosed in this closed space, forming the state shown in Fig.4. Part of Formulation A in the closed space was absorbed by the middle non-woven fabric layer through adhesive-free region 31 of the reticular adhesive layer 30, thereby being fixed in place.

Technical effect: the medical covering film can fix Formulation A on the affected skin during usage very well, the preparation is not easy to be squeezed off the target skin area by an external force, and can maintain the moisture in Formulation A very well. The skin covered by the Formulation A was anesthetized after about 90 minutes, and the Formulation A was removed from the skin 24 hours after the start of medication. The skin covered by the Formulation A was still anesthetized when the Formulation A was removed and one hour after the removal.

### Application Example 2

The usage method of the medical covering film of the Example 5 is as following: the patient's back skin had been suffering from postherpetic neuralgia for more than 8 years. The affected skin was a rectangle of about 15 cm×30 cm. A roll of 20 cm wide and 200 cm long medical covering film of Example 5 was cut for 35 cm long to obtain a piece of 20 cm×35 cm of the medical covering film. As shown in Fig.3, Formulation A was applied on a 16 cm×31 cm of region of the central region of the reticular adhesive layer 30 to form a layer with a thickness of 1 mm. The affected skin was covered with this medical covering film with Formulation A, so that the affected skin was completely covered by Formulation A. The 2 cm wide edge region of around the reticular adhesive layer 30 directly contacted the skin, and formed a flat rectangle closed space with the skin. The 16 cm×31 cm of Formulation A layer (that is, the water-containing semi-solid preparation layer 40 shown in Fig.4) in the middle was enclosed in this closed space, forming the state shown in Fig.4. Part of Formulation A in the closed space was adsorbed by the middle non-woven fabric layer through adhesive-free region 31 of the reticular adhesive layer 30, thereby being fixed in place.

Technical effect: After about 90 minutes, the patient's pain began to decrease significantly. The patient kept Formulation A and medical covering film on the skin for 18 hours. During the 18-hour medication period, the medical covering film maintained the moisture in Formulation A very well, and Formulation A was not removed from the original application place during her daily activities and sleep, because most of the local anesthetic preparation layer was adsorbed by the non-woven fabric layer at the original application place. In the next few months, she repeatedly used the Formulation A and B in the same way, and both achieved similar satisfactory results.

### Application Example 3

The usage method of the medical covering film of the Example 3 is as following: in order to reduce the pain caused by knee osteoarthritis, the medical staff applied a layer of the Formulation C on the knee joint of the patient to form a 15 cm wide loop around the knee joint, and the Formulation C was covered with the medical covering film of Example 3 (the edge region of the medical covering film, which is not in contact with the formulation, fixed the medical covering film and the formulation on the skin), Formulation C (the water-containing semi-solid preparation 40 shown in Fig.4) was enclosed in a closed space composed of the medical covering film and the skin. The combination of this covering film and the Formulation C was kept on the patient's knee joint 16 hours a day and used every day.

Technical effect: the anti-inflammatory drug diclofenac sodium in Formulation C was absorbed through the skin into the joint tissues. After two to three days, the patient felt a marked relief of pain. This is because the covering film prevented the evaporation of moisture in the formulation during the 16-hour application. Moreover, the stretchability of the covering film in all directions made the movement of the patient's joints unrestricted. The medical covering film can fix Formulation C to the affected skin during usage very well, the preparation is not easily squeezed off the target skin area by an external force, and the covering film can maintain the moisture in Formulation C very well.

### Application Example 4

A person's skin on the elbow is very dry. The medical staff applies the Formulation D with a thickness of about 2 mm to the dry skin, and covers the Formulation D with the medical covering film of Example 1-6 (the edge region of the medical covering film, which is not in contact with the formulation, fixes the medical covering film and the formulation on the skin), Formulation D (the water-containing semi-solid preparation 40 shown in Fig.4) is enclosed in a closed space composed of the medical covering film and the skin, forming the state shown in Fig.4.

Technical effect: the medical covering film can fix Formulation D which contains moisture and glycerin and other moisturizing components to the affected skin during usage very well, Formulation D is not easily squeezed off the target skin area by an external force, and can maintain the moisture in the Formulation D very well. The patient keeps Formulation D on the skin for 18 hours. This patient repeats the above-mentioned skin moisturizing procedure once a day, and the effect is very satisfactory.

### Application Example 5

The usage method of the medical covering film of the Example 8 is as following: in order to reduce the pain caused by knee osteoarthritis, the medical staff applies a layer of Formulation G on the knee joint of the patient to form a 15 cm wide loop around the knee joint, and the Formulation G is covered with the medical covering film of Example 8 (the edge region of the medical covering film that does not contact the formulation fixed the medical covering film and the formulation on the skin), Formulation G (the water-containing semi-solid preparation 40 shown in Fig.4) is enclosed in a closed space composed of the medical covering film and the skin. The combination of this covering film and Formulation G is kept on the patient's knee joint 16 hours a day and used every day. Because Formulation G contains polyvinyl alcohol and the adsorption layer of the medical covering film contains sodium borate. When the medical covering film covers Formulation G layer, sodium borate diffuses into the formulation layer and undergoes cross-linking reaction with the polyvinyl alcohol inside, solidifying and adhering Formulation G to the medical covering film. In this way, when the covering film is removed, Formulation G layer can be removed simultaneously, leaving no residual Formulation G on the skin.

Technical effect: The anti-inflammatory drug diclofenac sodium in Formulation G is absorbed through the skin into the joint tissues. After two to three days, the patient feels a marked relief of pain. This is because the covering film prevents the evaporation of moisture in the formulation during the 16-hour application. Moreover, the stretchability of the covering film in all directions makes the movement of the patient's joints without limitation. The medical covering film can fix the Formulation G to the affected skin during usage very well, Formulation D is not easily squeezed off the target skin area by external force, and the medical covering film can maintain the moisture in Formulation G very well.

Although the specific embodiments of the present disclosure are described above, those skilled in the art should understand that these are only illustrative examples, the scope of protection of the present disclosure is limited by the attached claims. Those skilled in the art can make various changes or modifications to these embodiments without deviating from the principle and essence of the present disclosure, but these changes and modifications all fall within the scope of protection of the present disclosure.

## Claims

1. A covering film, wherein, the covering film comprises a barrier film layer and an adsorption layer.

2. The covering film according to claim 1, wherein, the covering film is a medical covering film.

3. The covering film according to claim 1 or 2, wherein, the covering film contains a cross-linking agent; the cross-linking agent is preferably dispersed in the material of the adsorption layer;
preferably, the covering film contains a cross-linking agent higher than 0.01 mg/per square centimeter, more preferably higher than 0.1 mg/per square centimeter, or 0.01 mg-100 mg/per square centimeter, or 0.02-0.2 mg/per square centimeter;
preferably, the cross-linking agent is a substance containing boron element, glutaraldehyde, glyoxal, maleic acid, citric acid, trisodium trimetaphosphate, sodium hexametaphosphate, dianhydride, succinic acid, sulfosuccinic acid, or N,N'-methylenebisacrylamide; the substance containing boron element is preferably sodium borate;
more preferably, the covering film contains a substance containing boron element higher than 0.01 mg/per square centimeter, more preferably higher than 0.1 mg/per square centimeter, or 0.01 mg -100 mg/per square centimeter, or 0.02-2 mg/per square centimeter.

4. The covering film according to any one of claims 1-3, wherein, the covering film further comprises a reticular adhesive layer, one side of the adsorption layer is composited on the barrier film, and the reticular adhesive layer is composited on the other side of the adsorption layer; the area of the adhesive-free region of the reticular adhesive layer occupies 10% or more of the total area of the reticular adhesive layer, but not 100%;
or, the medical covering film further comprises a reticular adhesive layer, one side of the adsorption layer is composited on the barrier film, and the reticular adhesive layer is composited on the other side of the adsorption layer; the area of the adhesive-free region of the reticular adhesive layer occupies 10% or more of the total area of the reticular adhesive layer, but not 100%.

5. The covering film according to any one of claims 1 to 4, wherein, the material of the barrier film of the barrier film layer is polyethylene film, ethylene-vinyl acetate copolymer film or polyurethane film;
and/or, the adsorption layer is a material capable of adsorbing or retaining a water-containing semi-solid preparation, preferably a non-woven fabric;
and/or, the adhesive region of the reticular adhesive layer is an adhesive net composed of curves and/or straight lines;
and/or, the thickness of the reticular adhesive layer is 0.01 mm-0.75 mm, preferably 0.05 mm-0.75 mm, more preferably 0.25 mm-0.75 mm;
and/or, the adhesive region of the reticular adhesive layer is an adhesive net composed of a set of parallel longitude lines and a set of parallel latitude lines; preferably, the longitude lines and the latitude lines are perpendicular to each other; more preferably, the distance between two adjacent longitude lines is equal to the distance between two adjacent latitude lines; further more preferably, the width of each of the longitude lines and each of the latitude lines is 0.5 mm-1 mm;
and/or, the adhesive of the reticular adhesive layer is a medical pressure-sensitive adhesive that is insoluble in water, preferably silicone pressure-sensitive adhesive and/or acrylic pressure-sensitive adhesive;
and/or, one side of the adsorption layer is adhered to the barrier film layer by adhesive, or composited on the barrier film layer by hot pressing; the reticular adhesive layer is adhered to the other side of the adsorption layer by using spray coating method;
and/or, the area of the adhesive-free region of the reticular adhesive layer occupies 30% or more of the total area of the reticular adhesive layer; preferably, the area of the adhesive-free region of the reticular adhesive layer occupies 50% or more of the total area of the reticular adhesive layer; more preferably, the area of the adhesive-free region of the reticular adhesive layer occupies 70% or more of the total area of the reticular adhesive layer; further more preferably, the area of the adhesive-free region of the reticular adhesive layer occupies 70%-90% of the total area of the reticular adhesive layer.

6. The covering film according to claim 1, wherein, the length stretch rate of the covering film in all directions is more than 10%; preferably, the length stretch rate of the covering film in all directions is more than 10%-30%;
or, the length stretch rate of the medical covering film in all directions is more than 10%; preferably, the length stretch rate of the medical covering film in all directions is more than 10%-30%.

7. A system for fixing a preparation on the skin, wherein, the system comprises the covering film according to any one of claims 1-6 and a preparation layer for coating on the affected skin;
the covering film is used to cover the affected skin coated with the preparation layer in a way that the reticular adhesive layer orients towards the affected skin; the central region of the reticular adhesive layer is used for contacting the preparation layer; the edge region of the reticular adhesive layer is used to adhere to the skin area beyond the affected skin or the preparation layer, so as to fix the preparation layer on the affected skin or the skin beyond the affected skin, and the preparation layer is completely enclosed in a closed space composed of the medical covering film and the skin beyond the affected skin or the skin;
or, the medical covering film is used to cover the affected skin coated with the preparation layer in a way that the reticular adhesive layer orients towards the affected skin; the central region of the reticular adhesive layer is used for contacting the preparation layer; the edge region of the reticular adhesive layer is used to adhere to the skin area beyond the affected skin or the preparation layer, so as to fix the preparation layer on the affected skin or the skin beyond the affected skin, and the preparation layer is completely enclosed in a closed space composed of the medical covering film and the skin beyond the affected skin or the skin.

8. A system for fixing the preparation on the skin, wherein, the system comprises the covering film according to any one of claims 1-6;
wherein, the covering film comprises a barrier film layer and an adsorption layer, but does not comprise a reticular adhesive layer; the cross-linking agent is dispersed in the material of the adsorption layer; the system also comprises a preparation layer for coating on the affected skin; wherein, the preparation contains an adhesive agent and/or a cross-linkable substance; preferably, the preparation contains a cross-linkable substance and the covering film contains a corresponding cross-linking agent;
or the medical covering film comprises a barrier film layer and an adsorption layer, but does not comprise a reticular adhesive layer; the cross-linking agent is dispersed in the material of the adsorption layer; the system also comprises a preparation layer for coating on the affected skin; wherein, the preparation contains an adhesive agent and/or a cross-linkable substance; preferably, the preparation contains a cross-linkable substance and the medical covering film contains a corresponding cross-linking agent;
more preferably, the cross-linkable substance is polyvinyl alcohol, and the corresponding cross-linking agent is sodium borate;
further more preferably, the mass percentage of polyvinyl alcohol in the preparation is 0.2-30%, preferably 0.5-15%, more preferably 1%-5%;
more preferably, the cross-linkable substance is polyvinylpyrrolidone, and the corresponding cross-linking agent is N,N'- methylenebisacrylamide.

9. The system according to claim 7 or 8, wherein, the preparation is a water-containing semi-solid preparation;
preferably, the water-containing semi-solid preparation is a gel, a cream or a viscous liquid; preferably a skin nourishing preparation or a local anesthetic preparation;
and/or, the length of the outer edge of the covering film away from the outer edge of the water-containing semi-solid preparation layer is more than 5 mm, preferably 2 cm- 4 cm; or, the length of the outer edge of the medical covering film away from the outer edge of the water-containing semi-solid preparation layer is more than 5 mm, preferably 2 cm- 4 cm.

10. A usage method of the covering film according to any one of claims 1-6, wherein, the usage method comprises the following steps: applying a preparation on the central region of the reticular adhesive layer of the covering film, so that the edge region of the reticular adhesive layer is not coated by the preparation, to obtain the covering film with a preparation layer; then covering the covering film with the preparation layer on the affected skin, and making the preparation layer in contact with the affected skin, the edge region is adhered to or the skin not covered by the preparation layer, so as to fix the preparation layer on the affected skin or the skin beyond the affected skin, and the preparation layer is completely enclosed in a closed space composed of the covering film and the skin or the skin beyond the affected skin;
or, the usage method comprises the following steps: applying a preparation on the central region of the reticular adhesive layer of the medical covering film, so that the edge region of the reticular adhesive layer is not coated by the preparation, to obtain the medical covering film with a preparation layer; then covering the medical covering film with the preparation layer on the affected skin, and making the preparation layer in contact with the affected skin, the edge region is adhered to or the skin not covered by the preparation layer, so as to fix the preparation layer on the affected skin or the skin beyond the affected skin, and the preparation layer is completely enclosed in a closed space composed of the medical covering film and the skin or the skin beyond the affected skin;
preferably, the preparation is a water-containing semi-solid preparation, the preparation layer is a water-containing semi-solid preparation layer.

11. The usage method of the covering film according to claim 10, wherein, the usage method comprises the following steps: the covering film is covered on the affected skin coated with the preparation layer and surrounding skin in a way that the reticular adhesive layer orients towards the affected skin, the central region of the reticular adhesive layer contacts the preparation layer, the edge region of the reticular adhesive layer adheres to the skin beyond the affected skin or the skin not covered by the preparation layer, so as to fix the preparation layer on the affected skin or the skin beyond the affected skin, and the preparation layer is completely enclosed in a closed space composed of the covering film and the skin beyond the affected skin or the skin;
or, the medical covering film is covered on the affected skin coated with the preparation layer and surrounding skin in a way that the reticular adhesive layer orients towards the affected skin, the central region of the reticular adhesive layer contacts the preparation layer, the edge region of the reticular adhesive layer adheres to the skin beyond the affected skin or the skin not covered by the preparation layer, so as to fix the preparation layer on the affected skin or the skin beyond the affected skin, and the preparation layer is completely enclosed in a closed space composed of the medical covering film and the skin beyond the affected skin or the skin;
preferably, the preparation is a water-containing semi-solid preparation, the preparation layer is a water-containing semi-solid preparation layer.

12. The usage method of the covering film according to claim 10 or 11, wherein, the length of the outer edge of the covering film away from the water-containing semi-solid preparation layer or the outer edge of the preparation layer is more than 5 mm, preferably 2 cm-4 cm;
or, the length of the outer edge of the medical covering film away from the water-containing semi-solid preparation layer or the outer edge of the preparation layer is more than 5 mm, preferably 2 cm-4 cm.

13. A use of the covering film according to any one of claims 1-6 in the preparation of a medical device for treating herpes zoster pain, post-herpetic nerve damage pain, neuroma pain, phantom limb pain, diabetic peripheral neuralgia, joint pain, osteoarthritis pain, back pain, pain caused by gout, pain caused by soft tissue injury, postoperative incision pain, pain of broken skin caused by disease or trauma, burn pain or pain during burn scab removal; the covering film is, for example, a medical covering film.

14. A usage method of the system according to any one of claims 7-9 for fixing the preparation on the skin, wherein, the preparation is applied to the affected skin to form a preparation layer; and the covering film or the medical covering film is covered on the preparation layer, let the cross-linking agent and the cross-linkable substance undergo a cross-linking reaction to solidify the preparation layer.
